# EUROPÄISCHE PATENTSCHRIFT

(11) **EP 1 852 069 B1**
(45) Veröffentlichungstag und Bekanntmachung des Hinweises auf die Patenterteilung: **17.06.2015**
(21) Anmeldenummer: 06009200.4
(22) Anmeldetag: 04.05.2006
(51) Int. Cl.: A61B 5/151

(54) **Blutentnahmesystem zur Entnahme von Blut aus einem Körperteil für Diagnosezwecke**
System for sampling blood from a body part
Système de prélèvement sanguin d'une partie du corps

(43) Veröffentlichungstag der Anmeldung: 07.11.2007
(73) Patentinhaber: Roche Diagnostics GmbH, 68305 Mannheim (DE); F. Hoffmann-La Roche AG, 4070 Basel (CH)
(72) Erfinder: Weiss, Thomas, 68307 Mannheim (DE); List, Hans, 64754 Hesseneck-Kail-Bach (DE)
(74) Vertreter: Petirsch, Markus

(56) Entgegenhaltungen:
- EP-A- 1 625 825
- WO-A1-2005/110226
- US-A- 4 553 541
- US-A- 4 577 630
- US-B1- 6 332 871

## Beschreibung

Die Erfindung betrifft ein Blutentnahmesystem zur Entnahme von Blut aus einem Körperteil für Diagnosezwecke nach dem Oberbegriff des Anspruchs 1.

Für analytisch-diagnostische Zwecke wird eine geringe Menge Blut aus einem Körperteil entnommen. Dabei werden Lanzetten verwendet, die mit ihrer Spitze in dem Körperteil, vorzugsweise dem Finger oder dem Ohrläppchen, eine Wunde erzeugen. Diese Entnahme von Blut ist insbesondere als Selbstentnahme für die Diabetiker-Selbstkontrolle von großer Bedeutung, weil sie eine wesentlich verbesserte Einstellung der medikamentösen Behandlung von Diabetikern ermöglicht. Dadurch können schwere Spätfolgen der Diabetes vermieden werden.

Im Stand der Technik wurden deshalb eine Vielzahl von Geräten und Lanzettenvorrichtungen vorgeschlagen, bei denen der Einstichvorgang mechanisch durchgeführt wird. Dabei wird in der Regel eine Feder benutzt, die die Stechbewegung antreibt. Die Einstichtiefe kann durch einen Anschlag im Gehäuse definiert werden, gegen den die Lanzette oder ein Bauteil des Lanzettenantriebs anschlägt. Allerdings entsteht durch das Anschlagen eine erhebliche Erschütterung, die einen gleichmäßig sauberen Einstich verhindert.

Im Rahmen der Erfindung wurde festgestellt, dass bei den weitgehend gebräuchlichen Geräten, bei denen die Lanzette während der gesamten Stechbewegung, die sich aus Vortriebsphase und Rückführungsphase zusammensetzt, mit der antreibenden Feder gekoppelt ist, ein Nachschwingen der Feder dazu führt, dass die Spitze der Lanzette mehrfach in das Körperteil sticht. Dies ist jedoch unerwünscht, da dem Patienten zusätzliche Schmerzen zugefügt werden.

Dieses Problem besteht nicht bei aufwendigeren Konstruktionen, bei denen die Antriebsfeder nur während der Vortriebsphase mit der Lanzette gekoppelt und eine zusätzliche Mechanik vorgesehen sein, um die Lanzette aus dem Körperteil zurückzuführen.

Beispielsweise ist aus der US 6,206,901 B1 ist eine Blutentnahmevorrichtung mit einer Lanzette bekannt, bei der die Lanzette über einen Stößel und eine Antriebsfeder angetrieben wird. Dabei ist die Lanzette auf einem Teil ihrer Vortriebsphase von der Antriebsfeder entkoppelt. Zur Rückführung der Lanzette ist eine zweite Feder in der Nähe einer Austrittsöffnung des Gehäuses vorgesehen. Diese zweite Feder ist als Rückführungsfeder ausgebildet und dient dazu, die Lanzette nach dem Einstich wieder aus der Wunde zurückzuführen. Die Rückführfeder wird erst während der Vortriebsphase durch die Lanzette gespannt. Die kinetische Energie der Lanzette wird in der Feder gespeichert und nach Erreichen der maximalen Spannung der Rückführfeder von dieser an die Lanzette zurückgegeben. Dies hat jedoch den Nachteil, dass die Lanzette durch die Rückführfeder abgebremst wird. Dadurch ist der Eintritt der Lanzette in die Haut des Patienten langsam und schmerzhaft.

Aus der EP 0 036 443 B1 ist ebenfalls eine Lanzettenvorrichtung bekannt, bei der eine Lanzette über eine Antriebsfeder während der Vortriebsphase angetrieben wird und die Antriebsfeder während eines Teils der Stechbewegung von der Antriebsfeder entkoppelt ist. Zur Rückführung der Lanzette nach dem Einstich ist eine zweite Feder im Bereich einer Öffnung des Gehäuses der Lanzettenvorrichtung vorgesehen, die die Lanzette nach dem Einstich zurück bewegt. Die zweite Feder wird ebenfalls erst durch die Lanzette während der Vortriebsphase der Stechbewegung gespannt. Dadurch wird die Lanzette abgebremst; ihre kinetische Energie wird in Federenergie umgewandelt, bis die kinetische Energie vollständig aufgezehrt ist. Dabei wird wiederum der Einstich der Lanzette in das Körperteil verlangsamt.

In der US 2004/0267300 A1 ist eine Lanzettenvorrichtung beschrieben, bei der die Lanzette über eine rotierende Feder, die mehrere Federarme hat, angetrieben wird. Dazu werden die Federarme gespannt. Sie bewegen nach Lösen einer Arretierung die Lanzette in Richtung einer Gehäuseöffnung. Nach dem Entspannen der Federarme, die zwischen einem äußeren Ring und einem inneren Ring ausgebildet sind, findet kein Zurückschwingen statt. Um die Lanzette aus ihrer Einstechposition zurückzubewegen, sind Rückholfederarme vorgesehen, die zwischen dem inneren Ring und einer Innenachse angeordnet sind. Die Rückholfederarme werden vor Beginn der Stechbewegung gespannt. Sobald die Lanzette in ein Körperteil eingestochen hat, also die Federarme vollständig entspannt sind, werden auch die Rückholfederarme entspannt, so dass die Lanzette aus ihrer Einstichposition zurückgeholt wird. Da die Rückholfederarme schon vorgespannt sind, wird keine kinetische Energie der Lanzette während der Stechbewegung zum Spannen der Rückholfeder verwendet. Der Einstich erfolgt also schnell und damit wenig schmerzhaft. Allerdings ist die Mechanik der Lanzettenvorrichtung sehr aufwendig, da ein exaktes Zusammenwirken der spannenden Federarme wie auch der Rückholfederarme gewährleistet sein muß. Weiterhin muß die Rotationsbewegung der Federn in eine translatorische Bewegung der Lanzette umgesetzt werden.

Eine Blutentnahmevorrichtunge gemäß dem Oberbegriff von Anspruch 1 ist aus der US 4,553,541 bekannt.

Alle diese bekannten Blutentnahmevorrichtungen sind konstruktiv sehr viel aufwendiger als die einleitend genannten Konstruktionen, bei denen die Feder (üblicherweise eine einfache Schraubenfeder) eine linear in Einstichrichtung wirkende Entspannungsbewegung ausführt, die direkt auf die Lanzette übertragen wird. Dieses Konstruktionsprinzip ist nicht nur einfach und damit kostengünstig, sondern auch platzsparend. Es ermöglicht deswegen eine schlanke Handbauweise, die die Handhabung erleichtert. Um einen schnellen und damit relativ schmerzfreien Einstich der Lanzette in einer Körperteil zu verwirklichen, werden starke Antriebsfedern verwendet. Dabei ist die Gefahr, dass durch das Nachschwingen der Antriebsfeder die Lanzette bzw. die Lanzettennadel mehrfach in das Körperteil einsticht, besonders groß. Wird die Antriebsfeder entsprechend schwächer ausgelegt, so ist die maximale Stechtiefe in der Haut nicht gewährleistet. Es wird dann nicht genug Blut gewonnen.

Der Erfindung liegt die Aufgabe zugrunde, ein möglichst einfaches Blutentnahmesystem vorzuschlagen, das zugleich einen schnellen und schmerzarmen Einstich gewährleistet.

Die gestellte Aufgabe wird durch ein Blutentnahmesystem gemäß Anspruch 1 gelöst.

Das Blutentnahmesystem umfaßt ein Gehäuse mit einer Lanzettenführung, von der eine Lanzette auf einem vorbestimmten Einstichweg geführt wird, und einem Lanzettenantrieb zum Antreiben einer Stechbewegung der Lanzette auf dem vorbestimmten Einstichweg. Die Stechbewegung schließt eine Vortriebsphase in Einstichrichtung und eine sich an einen Umkehrpunkt anschließende Rückführungsphase entgegen der Einstichrichtung ein. Der Lanzettenantrieb hat eine Antriebsfeder, die hinter der Lanzette angeordnet ist und während der Vortriebsphase mit der Lanzette so gekoppelt ist, dass die Entspannungsbewegung der Antriebsfeder während mindestens eines Teils der Vortriebsphase der Stechbewegung die Lanzette direkt (also ohne Getriebeelemente) antreibt. Am Ende der Vortriebsphase dringt die Spitze der Lanzette in das Körperteil ein, um eine Wunde zu erzeugen. Nach Erreichen des Umkehrpunkts wird die Lanzette in der Rückführungsphase der Stechbewegung aus der Haut herausgezogen. Bei diesen Blutentnahmesystemen, bei denen die Antriebsfeder hinter der Lanzette, insbesondere koaxial hinter der Lanzette angeordnet ist, bildet die Lanzette mit einer oder mehreren auf sie einwirkenden Feder ein schwingungsfähiges Gesamtsystem. Während der Rückführungsphase der Lanzette befindet sich die Antriebsfeder im Rückführungsweg der Lanzette, so dass die Lanzette auf die Antriebsfeder wirken kann. Dadurch kann Energie der Lanzette wieder auf die Antriebsfeder übertragen und in ihr gespeichert werden. Die dadurch erneut gespannte Antriebsfeder kann die Lanzette wieder in Einstichrichtung treiben, so dass die Gefahr des Mehrfacheinstichs besteht. Erfindungsgemäß ist bei einem solchen System eine Schwingungskontrolleinrichtung vorgesehen, dass während der Rückführungsphase der Stechbewegung auf die Lanzette einwirkt. Die Schwingungskontrolleinrichtung wird nachfolgend auch mit der Kurzbezeichnung "ROTOCOM" (Retraction Operating Ozilation Control Means) bezeichnet.

Die Schwingungskontrolleinrichtung kann im wesentlichen auf drei Arten auf das Schwingungsverhalten der Lanzette einwirken. Zum Einen kann sie einen verschiebbaren Anschlag aufweisen, so dass die Schwingungsbewegung der Lanzette in Einstichrichtung durch den Anschlag begrenzt wird. Der Anschlag beschränkt die Schwingung, die schneller zum Stillstand kommt. Als zweite Möglichkeit kann eine Bedämpfung der Schwingung stattfinden, so dass die Schwingungsamplitude sehr stark abnimmt. Im Idealfall kommt die Lanzette schon während der ersten Rückführung zum Stillstand. Als dritte Möglichkeit kann eine Ortsverschiebung der Antriebsfeder vorgesehen sein, durch die der Nullpunkt der Schwingung, um den die Antriebsfeder schwingt, entgegen der Einstichrichtung derart verschoben wird, dass auch bei maximaler Auslenkung der Antriebsfeder ein Einstechen der Lanzette in das Körperteil ausgeschlossen ist. Die Schwingung selbst wird nicht beeinflußt. Ein mehrfaches Hin- und Herschwingen ist möglich.

Allen drei Ausführungsformen ist gemeinsam, dass das Schwingungsverhalten der Lanzette durch die Schwingungskontrolleinrichtung so kontrolliert wird, dass ein mehrfacher Einstich der Lanzette in das Körperteil verhindert wird.

Durch das (vorzugsweise im wesentlichen nur) während der Rückzugsphase auf die Lanzette einwirkende ROTOCOM-System wird das Mehrfachstechen in die Haut verhindert, während gleichzeitig die mit der koaxialen Anordnung der Antriebsfeder hinter der Lanzette verbundenen Vorteile, insbesondere hinsichtlich einer einfachen Konstruktion, erhalten bleiben.

Im Rahmen der Erfindung wurde festgestellt, dass die Verwendung des ROTOCOM-Systems zu weiteren bedeutenden Vorteilen führt:

Das ROTOCOM-System wirkt während der Rückführungsphase auf die Lanzette ein, nicht jedoch während der Vortriebsphase. Damit bleibt die Vortriebsphase unbeeinflußt. Die Lanzette kann folglich mit hoher Geschwindigkeit in das Körperteil einstechen, so dass der Patient nur relativ geringe Schmerzen empfindet.

Die Einstellung der Einstechtiefe der Lanzette in das Körperteil ist unabhängig von der Schwingungskontrolleinrichtung. Es müssen keine zusätzlichen konstruktiven Maßnahmen ergriffen werden, um eine mögliche Beeinflussung auszuschließen oder zu kompensieren.

Aufwendige Konstruktionen, die eine Entkopplung der Antriebsmechanik von der Lanzette während der Rückführungsphase vorsehen, und die deshalb eine gesonderte Rückführungsmechanik aufweisen müssen, können durch konstruktiv einfache und daher kostengünstige Systeme ersetzt werden, bei denen die Lanzette getriebefrei direkt von der Feder angetrieben wird, insbesondere während der gesamten Stechbewegung, also während der Vortriebsphase und der Rückführungsphase, mit der Feder gekoppelt ist.

Gemäß einer bevorzugten Ausführungsform umfasst die Schwingungskontrolleinrichtung zur Verhinderung des Mehrfacheinstechens einen bewegbaren Endanschlag. Der Endanschlag wird nach Erreichen des Umkehrpunkts der Stechbewegung der Lanzette entgegen der Einstichrichtung in eine Halteposition verschoben. Dadurch wird bei einem Nachschwingen der Weg der Lanzette in Einstichrichtung begrenzt. Der bewegbare Endanschlag ist vor Beginn des Einstichs in einer Ausgangsposition gehalten, in der er ohne Einwirkung auf den Einstichvorgang ist und die Stechbewegung nicht beeinflußt. Erst nach Erreichen des Umkehrpunkts der Stechbewegung wird der Endanschlag aus der Ausgangsposition in die Halteposition gebracht, in der er bei einem erneuten Schwingen der Lanzette in Einstichrichtung auf die Lanzette einwirkt und ihren Weg in Einstichrichtung begrenzt.

Vorzugsweise ist der Endanschlag in der Halteposition fixiert. Dadurch wird eine zuverlässige Begrenzung der Bewegung der Lanzette erreicht; ein Verschieben des Endanschlags durch die sich erneut in Einstichrichtung bewegende Lanzette ist durch die Arretierung des Endanschlags ausgeschlossen. Sie kann beispielsweise durch einen Haken realisiert werden.

Um den Endanschlag in die Halteposition zu verschieben, ist in einer bevorzugten Ausführungsform eine Anschlagfeder vorgesehen. Die Anschlagfeder bewegt den Endanschlag aus seiner Ausgangsposition in die Halteposition. Dazu wird die Anschlagfeder vorgespannt und der Endanschlag in seiner Ausgangsposition arretiert. Die Anschlagfeder ist also während der Stechbewegung in Einstichrichtung der Lanzette bereits vorgespannt. Die Arretierung der Anschlagfeder wird bevorzugt am Ende der Vortriebsphase der Stechbewegung gelöst. Der Endanschlag wird entgegen der Einstichrichtung bewegt. Bei dieser Bewegung, bei der die Federenergie in kinetische Energie umgewandelt wird, kann auch die Lanzette während der Rückführungsphase zusätzlich angetrieben werden. Dadurch kann die Rückführungsphase beschleunigt werden.

Vorzugsweise ist bei dem erfindungsgemäßen Blutentnahmesystem eine Spannvorrichtung zum Spannen der Anschlagfeder vorgesehen. Die Spannvorrichtung weist bevorzugt eine Gewindestange mit Gewinde und ein Spannelement auf. Durch Drehen der Gewindestange wird das Spannelement entlang der Stange bewegt und nimmt die Anschlagfeder mit. Dabei wird das Spannelement während seiner Spannbewegung an einer Führungsfläche geführt. Durch die Führungsfläche wird zuverlässig verhindert, dass sich das Spannelement mit der Gewindestange mitdreht. Auf diese Weise wird die Drehbewegung der Gewindestange in eine translatorische Bewegung umgewandelt, so dass die Anschlagfeder, die vorzugsweise eine Spiralfeder ist, aus ihrer Ruheposition herausgedehnt bzw. zusammengedrückt wird. Die durch das Spannelement und die Gewindestange aufgebrachte Energie wird in der Feder gespeichert. Die Feder wird dann in gespanntem Zustand arretiert, so dass die gespeicherte Energie der Feder erhalten bleibt. Erst nach Lösen der Arretierung wird die Federenergie wieder in kinetische Energie transformiert.

Die Führungsfläche zum Führen des Spannelements kann als Kante ausgebildet sein, die sich parallel zur Gewindestange erstreckt. Alternativ kann die Führungsfläche beispielsweise als Nut oder Absatz im Gehäuse vorgesehen sein.

Nach Erreichen der Ausgangsposition der Anschlagfeder, in der sie gespannt ist, wird das Spannelement beim Bewegen entgegen der Bewegrichtung zum Spannen an einer Rückleitkante geführt. Die Führung an der Rückleitkante erfolgt derart, dass das Spannelement mit der Anschlagfeder außer Eingriff kommt. Dazu wird das Spannelement in eine Position gedreht, in der es an der Rückleitkante anliegt. In dieser Position kann sich die Anschlagfeder nach Lösen der Arretierung bewegen, ohne mit dem Spannelement zu kollidieren.

Wenn das Blutentnahmesystem als sogenanntes Single Use Device, also als Einwegstechvorrichtung, vorgesehen ist, kann die Anschlagfeder bereits bei der Montage vorgespannt und im vorgespannten Zustand eingebaut werden. Die Anschlagfeder bzw. der vorgespannte Endanschlag muß dann lediglich im Umkehrpunkt der Stechbewegung gelöst werden, so dass der Endanschlag entgegen der Einstichrichtung bewegt wird. Auf eine Spannvorrichtung kann in diesem Fall verzichtet werden.

Gemäß einer weiteren bevorzugten Ausführungsform weist das ROTOCOM-System eine Dämpfungseinrichtung auf, durch das die Bewegung der Lanzette in der Rückführungsphase abgebremst wird. Die Lanzette führt folglich während der Rückführungsphase eine gedämpfte Bewegung aus. Als Dämpfung wird eine Abnahme der Amplitude einer Schwingung (hier des Nachschwingens der Lanzette) bezeichnet, die durch einen Energieverlust verursacht wird. Der Energieverlust ist dabei der Übergang der Schwingungsenergie in eine andere Energieform. Die kinetische Energie der Lanzette wird durch die Dämpfungseinrichtung schnell in eine andere Energieform umgewandelt, so dass die Lanzette während der Rückführungsphase im Idealfall sehr rasch bis zum vollständigen Stillstand abgebremst und ein Nachschwingen vollständig verhindert wird. Dabei muß darauf geachtet werden, dass die Lanzette entgegen der Einstichrichtung wenigstens so weit bewegt wird, dass die Lanzettenspitze aus der Wunde in dem Körperteil entfernt wird.

Bevorzugt ist die Dämpfungseinrichtung als pneumatischer Dämpfer ausgeführt. Der Dämpfer kann beispielsweise mit einem Kolben und einer Druckkammer ausgestattet sein, wobei während der Rückführungsphase der Lanzette der Kolben die in einer Druckkammer vorhandene Luft komprimiert und die Luft über die Drosselstelle abgeblasen wird, so dass dem System Energie entzogen wird.

Dazu ist bevorzugt ein Kopplungselement zwischen der Lanzette und der Antriebsfeder vorgesehen. Das Kopplungselement wird in einem Zylinder geführt, der beispielsweise ein Bestandteil des Gehäuses des Blutentnahmesystems sein kann. An dem Kopplungselement sind bevorzugt Dichtlippen angeordnet, die an der Innenwand des Zylinders anliegen. Damit arbeitet das Dämpfungssystem nach dem Prinzip einer Luftpumpe, wobei die Dichtlippen des Kopplungselements derart angeordnet sind, dass sie bei der Bewegung des Kopplungselements in einer Richtung Luft vorbeiströmen lassen, in der entgegengesetzten Richtung jedoch nicht. Die Verwendung der Dichtlippen erweist sich als sehr einfaches Prinzip, ist jedoch sehr effizient. Dichtlippen können darüber hinaus sehr einfach an dem Kopplungselement angebracht werden, beispielsweise bei einem im Querschnitt runden Kopplungselement mittels einer Nut.

In einer weiter bevorzugten Ausführungsform liegen die Dichtlippen des Kopplungselements an der Innenwand des Zylinders derart an, dass die Lanzette nicht wesentlich gebremst wird (das heißt, dass die Stechbewegung der Lanzette in der Vortriebsphase nicht in einem praktisch störenden Ausmaß beeinflußt wird). Die Lanzette kann mit hoher Geschwindigkeit in das Körperteil eindringen.

Durch geeignete Anordnung der Dichtlippen des Kopplungselements wird die im Zylinder enthaltende Luft während der Rückführungsphase der Lanzette vor den Dichtlippen, komprimiert. Dadurch wird die Lanzette in ihrer Rückführungsphase abgebremst. Ein Nachschwingen wird effektiv verhindert, indem bei der Kompression entweder die Kompressionswärme abgeführt wird, beispielsweise über die Zylinderwände, und/oder über eine Drosselstelle die komprimierte Luft abgeblasen wird. Außerdem werden die Dichtlippen von steigendem Luftdruck an die Zylinderwand angepresst und verursachen so eine erhöhte Reibung. Das Abbremsen der Lanzette kann durch Wahl des Neigungswinkels der Dichtlippen gegenüber der Zylinderwand so optimiert werden, dass der während der Rückführungsphase von der Lanzette zurückgelegte Weg vorbestimmt werden kann. Gleichzeitig kann durch den Neigungswinkel auch festgelegt werden, wie groß der Einfluß auf die Lanzette während der Vortriebsphase der Stechbewegung ist. Durch geeignete Wahl des Materials der Dichtlippen, deren Härte und Elastizität, kann das Dämpfungsverhalten des Dämpfungssystems festgelegt sein.

Anhand mehrerer in den Figuren dargestellter Ausführungsbeispiele wird die Erfindung nachstehend näher erläutert. Es zeigen:
- Fig. 1: ein erfindungsgemäßes Blutentnahmesystem mit einem Endanschlag während der Vortriebsphase einer Stechbewegung einer Lanzette;
- Fig. 2: das Blutentnahmesystem aus Figur 1 während der Rückführungsphase der Stechbewegung;
- Fig. 3: eine alternative Ausführungsform des Blutentnahmesystems mit einer Gewindestange zum Bewegen des Endanschlags in eine Ausgangsposition und zum Spannen einer Antriebsfeder der Lanzette;
- Fig. 4: eine Detailzeichnung im Querschnitt durch die Gewindestange aus Figur 3;
- Fig. 5: eine schematische Prinzipskizze einer alternativen Spannvorrichtung für die Lanzette und den Endanschlag;
- Fig. 6a: ein Schnittbild durch eine alternative Ausführung eines Blutentnahmesystems mit einer Dämpfungseinrichtung umfassend einen pneumatischen Dämpfer;
- Fig. 6b: einen vergrößerten Ausschnitt aus Fig. 6a; und
- Fig. 6c: einen vergrößerten Detailausschnitt aus Fig. 6a; und
- Fig. 7: eine Prinzipskizze eines Kopplungselements für eine Lanzette mit einem hydraulischen Dämpfer.

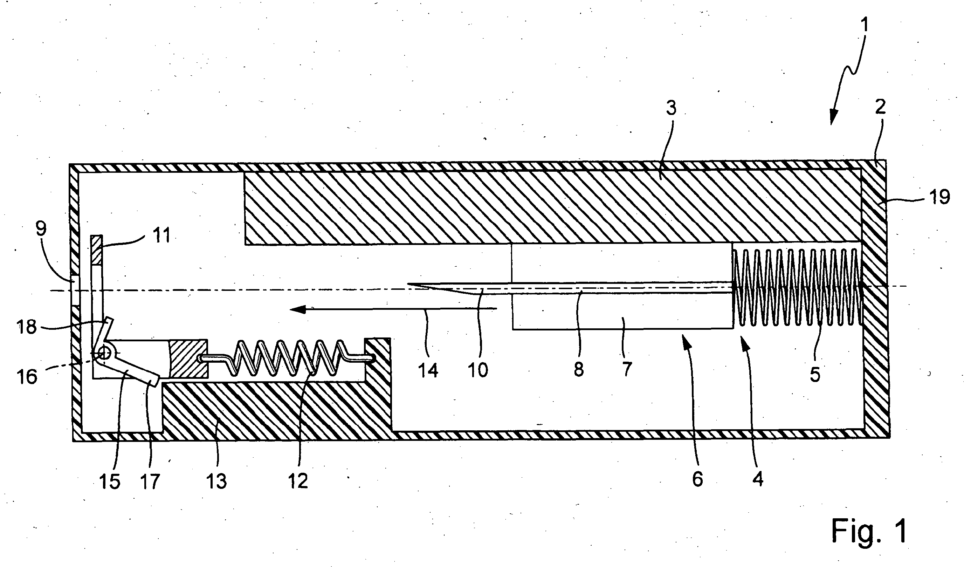

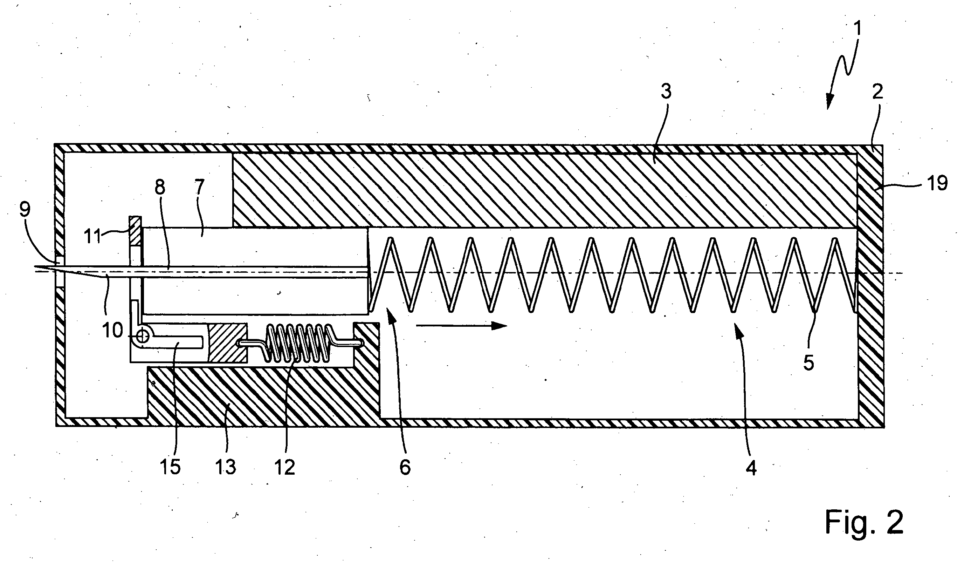

Die Figuren 1 und 2 zeigen eine Ausführungsform eines Blutentnahmesystems 1 im Längsschnitt. Das Blutentnahmesystem 1 besteht im wesentlichen aus einem Gehäuse 2 mit einer Lanzettenführung 3 und einem mit dem Gehäuse 2 gekoppelten Lanzettenantrieb 4. Der Lanzettenantrieb 4 umfaßt eine Antriebsfeder 5 und ein Kopplungselement 6, das als Lanzettenhalter 7 ausgebildet ist. In dem Lanzettenhalter 7 wird eine Lanzette 8 gehalten.

Das Gehäuse 2 weist an einer Stirnseite eine Öffnung 9 auf, durch die die Lanzette 8 aus dem Gehäuse 2 heraustreten kann, und eine Wunde in einem Körperteil erzeugt. Alternativ kann die Öffnung 9 auch größer ausgebildet sein, so dass sich die Haut eines Körperteils, an dem das Gehäuse 2 anliegt, in die Öffnung 9 hineinwölbt. Die Lanzette 8 muß dann nicht aus dem Gehäuse 2 heraustreten, um eine Wunde zu erzeugen.

Das Gehäuse 2 umfaßt weiter einen Endanschlag 11, der über eine Anschlagfeder 12 mit einer Anschlaghalterung 13 gekoppelt ist. Die Anschlaghalterung 13 ist fest mit dem Gehäuse 2 verbunden. Sie führt gleichzeitig den Endanschlag 11.

In Figur 1 ist das Blutentnahmesystem 1 während der Vortriebsphase der Stechbewegung der Lanzette gezeigt. Die Antriebsfeder 5 war vorgespannt und bewegt nun die Lanzette 8 in Einstichrichtung, die durch den Pfeil 14 dargestellt ist.

Der Endanschlag 11 ist in seiner Ausgangsposition dargestellt, bei der eine als Klinke 15 ausgebildete Arretierung den Endanschlag 11 in seiner Ausgangsposition hält. Die Klinke 15 ist drehbar um ein Drehlager 16 an dem Endanschlag 11 befestigt. Sie ist L-förmig ausgebildet und weist einen langen L-Schenkel 17 und einen kurzen L-Schenkel 18 auf. Der lange L-Schenkel 17 liegt in seinem freien Ende an der Anschlaghalterung 13 so an, dass der Endanschlag 11 arretiert ist. Die Anschlagfeder 12 ist vorgespannt. Bevorzugt ist sie, wie im Beispiel gezeigt, als Zugfeder ausgebildet.

Während der Vortriebsphase der Stechbewegung wird die Lanzette 8 in Richtung auf die Öffnung 9 zu, also in Einstichrichtung, bewegt. Am Ende der Vortriebsphase schlägt die Lanzette 8 an den kurzen L-Schenkel 18 in der Klinke 15 an und dreht in die Klinke 15 entgegen dem Uhrzeigersinn. Dadurch kommt der lange L-Schenkel 17 außer Eingriff mit der Anschlaghalterung 13, so dass der Endanschlag 11 nicht mehr arretiert ist. In diesem Moment hat die Lanzette 8 in ihrer Stechbewegung den Umkehrpunkt erreicht und wird von der Antriebsfeder 5 wieder in Richtung Gehäuseende 19 gezogen.

Gleichzeitig wird während der Rückführungsphase der Stechbewegung der Lanzette 8 der Endanschlag 11 aus seiner Ausgangsposition entgegen der Einstichrichtung bewegt (Figur 2). Dabei zieht sich die gespannte Anschlagfeder 12 zusammen, so dass der Endanschlag 11 entgegen der Einstichrichtung bewegt wird, bis der Endanschlag 11 seine Halteposition erreicht hat.

Während der Bewegung des Endanschlags 11 liegt der Endanschlag 11 bevorzugt so an der Lanzette 8 an, dass die Lanzette 8 in der Rückführungsphase der Stechbewegung nicht oder nicht nur von der Antriebsfeder 5, sondern (auch) von der Anschlagfeder 12 indirekt bewegt wird. Dadurch kann die Rückführungsgeschwindigkeit unabhängig vom Vortrieb gestaltet werden bzw. die Lanzette 8 beschleunigt werden.

Sobald der Endanschlag 11 seine Halteposition erreicht hat, wird er vorzugsweise von der Restspannung der Anschlagfeder 12 auf einen Anschlag an der Führung 13 gedrückt, so dass er einen festen Anschlag für die Lanzette 8 nach vorne bildet. Damit wird verhindert, dass die Lanzette 8, die noch mit der Antriebsfeder in Wechselwirkung steht oder diese wieder aufnimmt, weiter als bis zum Endanschlag vorschwingen kann. Der Endanschlag 11 ist dann in seiner Halteposition fixiert. Die Lanzette 8 wird dann nur noch durch die Antriebsfeder 5 (entgegen der Einstichrichtung) bewegt.

Alternativ wird der Endanschlag 11 elastisch in seiner Halteposition gehalten; er ist nicht fixiert, sondern wird lediglich von der Anschlagfeder 12 gehalten. Somit kann der Endanschlag 11 mit einer kleinen Amplitude, um die Halteposition herum schwingen.

Vorzugsweise ist die Anschlagfeder 12 vorgespannt; insbesondere ist sie noch vorgespannt, wenn der Endanschlag 11 in seiner Halteposition verschoben ist. Besonders bevorzugt weißt die Anschlagfeder 12 eine hohe Vorspannkraft auf, die beispielsweise durch eine hohe Federkonstante oder bei einer geringen Federkonstante durch eine große Auslenkung erzielt werden kann. Dann kann sich der Endanschlag 11 nur mit einer kleinen Amplitude um seine Halteposition bewegen, auch wenn die Lanzette 8 von der Antriebsfeder 12 beim Nachschwingen wieder in Einstichrichtung bewegt wird und auf den Endanschlag 11 eine Kraft in Richtung der Öffnung 9 ausübt.

In der Halteposition des Endanschlags 11 muß dessen Abstand von der Öffnung 9 größer sein als die Länge der Spitze 10 der Lanzette 8, so dass die Spitze 10 nicht durch die Öffnung 9 des Gehäuses 2 nach außen tritt. Vorzugsweise ist der Abstand zwischen Endanschlag 11 und Öffnung 9 in der Halteposition so groß, dass die Lanzettenspitze trotz des Schwingens des Endanschlags 11 um die Halteposition (mit einer kleinen Amplitude, die durch die Federkonstante der Anschlagfeder 12 bestimmt ist) nicht aus der Öffnung 9 heraustritt.

Durch die Ausgangsposition des Endanschlags 11 kann die Einstichtiefe der Lanzette 8 variabel eingestellt werden. Dadurch kann er gleichzeitig zur Einstellung der Einstechtiefe der Spitze 10 der Lanzette 8 dienen.

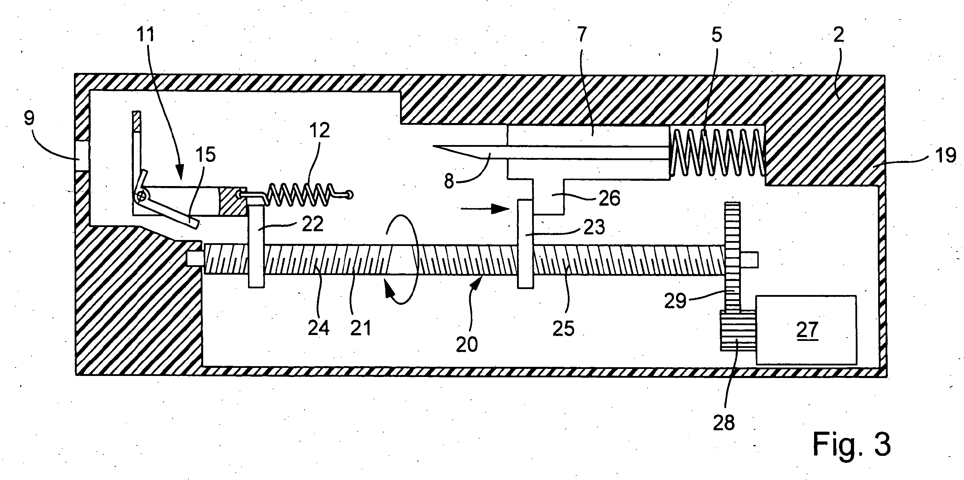

Figur 3 zeigt eine alternative Ausführungsform eines Blutentnahmesystems mit einem Gehäuse 2 und einer Öffnung 9 im Prinzipbild. Darin ist eine erste Ausführungsform einer Spannvorrichtung 20 für das Spannen der Antriebsfeder 5 und der Anschlagfeder 12 dargestellt. Sie umfaßt eine Gewindestange 21 mit einem ersten Spannelement 22 zum Spannen der Anschlagfeder 12 und einem zweiten Spannelement 23 zum Spannen der Antriebsfeder 5. Die Gewindestange 21 weist zwei gegenläufig ausgebildete Gewinde 24,25 auf, wobei das erste Gewinde 24 das erste Spannelement 22 und das zweite Gewinde 25 das zweite Spannelement 23 bewegt.

In dem in Figur 3 gezeigten Beispiel wird durch Drehen der Gewindestange 21 im Uhrzeigersinn das Spannelement 22 in Richtung Öffnung 9 des Gehäuses 2 bewegt, während gleichzeitig das Spannelement 23 in Richtung Gehäuseende 19 bewegt wird. Während der Bewegung der Gewindestange 21 greift das erste Spannelement 22 direkt an dem Endanschlag 11 an und bewegt diesen aus seiner Halteposition in seine Ausgangsposition. Dabei rastet die Klinke 15 an der Anschlaghalterung 13 ein, so dass der Endanschlag 11 in seiner Ausgangsposition gehalten wird. Das zweite Spannelement 23, das von dem Gewinde 25 bewegt wird, greift an einen Mitnehmer 26 an dem Lanzettenhalter 7 an. Auf diese Weise wird der Lanzettenhalter 7 mit der Lanzette 8 entgegen der Einstichrichtung verschoben und die Antriebsfeder 5 gespannt. Auch hier ist eine nicht dargestellte Arretierung vorgesehen, die die Lanzette 8 in ihrer gespannten Position hält. Wenn diese Arretierung gelöst wird, beginnt die Stechbewegung der Lanzette 8, durch die sie in Einstichrichtung bewegt wird.

Die Klinke 15 zeigt nur eine Möglichkeit, um den Endanschlag 11 in seiner Ausgangsposition zu arretieren bzw. aus dieser Position zu lösen. Selbstverständlich sind auch beliebige andere Arretierungen möglich, die den Endanschlag 11 in seiner Ausgangsposition fixieren. Insbesondere kann eine Arretierung vorgesehen sein, die erst nach Zurücklegen eines bestimmten Weges während der Rückführungsphase der Stechbewegung durch die Lanzette 8 oder den Lanzettenhalter 7 gelöst wird, so dass sich der Endanschlag 11 auch noch zu Beginn der Rückführungsphase der Stechbewegung der Lanzette 8 in seiner Ausgangsposition befindet.

Um der Möglichkeit Rechnung zu tragen, dass die Antriebsfeder 5 und die Anschlagfeder 12 unterschiedliche Federkonstanten und Größen aufweisen und folglich die Spannwege des Endanschlags 11 und des Lanzettenhalters 7 unterschiedlich sind, können die beiden Gewinde 24 und 25 unterschiedliche Steigungen und unterschiedliche Längen haben.

Die Gewindestange 21 kann mechanisch durch eine Kurbel oder einen anderen Mechanismus angetrieben werden oder elektrisch, wie in Figur 3 gezeigt. Ein Motor 27 treibt über ein Zahnrad 28 ein zweites Zahnrad 29 an, das mit der Gewindestange 21 starr gekoppelt ist. Die Drehung des Motors 27 in eine Richtung bewirkt eine entsprechende Drehung der Gewindestange 21 und somit ein Verschieben der Spannelemente 22 und 23.

Nachdem die Antriebsfeder 5 und die Anschlagfeder 12 durch die Spannvorrichtung 20 gespannt sind, können die Spannelemente 22 und 23 wieder in Richtung Mitte der Gewindestange 21, also aufeinander zu, bewegt werden. Dazu ist genau wie bei der Spannbewegung eine Führung der Spannelemente 22 und 23 notwendig. Die Führung während der Spannbewegung wird durch eine Führungsfläche 30 verwirklicht, an der die Spannelemente anliegen; das Rückführen bzw. Bewegen der Spannelemente entgegen der Bewegrichtung zum Spannen wird durch eine Rückleitkante 31 geleitet.

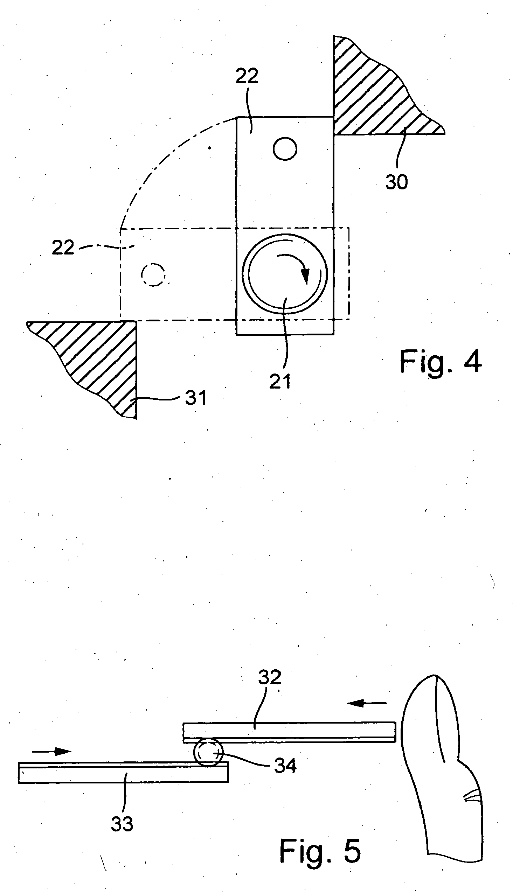

Figur 4 zeigt die Gewindestange 21 im Querschnitt mit einem Spannelement 22 in zwei Positionen. In der gestrichelt dargestellten Position des Spannelementes 22 liegt das Spannelement 22 an der Rückleitkante 31 an. Durch Bewegen der Gewindestange entgegen des Uhrzeigersinns dreht das Spannelement 22 so lange mit, bis es an der Rückleitkante 31 anliegt. Anschließend wird es entlang der Rückleitkante 31 und der Gewindestange 21 verschoben. In dieser Position befindet sich das Spannelement 22 nicht im Bewegungsweg des Endanschlags 11.

Durch Drehen der Gewindestange in Uhrzeigerrichtung wird das Spannelement 22 ebenfalls in Uhrzeigerrichtung mitgedreht, bis es an der Führungsfläche 30 anschlägt. Ein weiteres Drehen der Gewindestange 21 bewirkt, dass das Spannelement 22 entlang der Führungsfläche 30 eine translatorische Bewegung in die Zeichnungsebene hinein ausführt. Durch diese translatorische Bewegung wird das Spannen der in Figur 3 gezeigten Anschlagfeder 12 des Endanschlags 11 durchgeführt.

Das in Figur 4 dargestellte Prinzipbild mit dem Spannelement 22 gilt auch für das Spannelement 23. Das Spannelement 23 wird ebenfalls entlang der Führungsflächen 30 bzw. der Rückleitkante 31 während der Bewegung der Gewindestange 21 geführt. Nach dem Spannen der Antriebsfeder 5 des Lanzettenhalters 7 liegt das Spannelement 23 an der Rückleitkante 31 an, wenn es in Richtung Mitte der Gewindestange 21 bewegt wird. In dieser Position des Spannelementes 23 kann die Lanzette 8 mit dem Lanzettenhalter 7 und dem Mitnehmer 26 die Stechbewegungen in Einstichrichtung ausführen, ohne dass der Mitnehmer 26 gegen das Spannelement 23 stößt.

In Figur 5 ist eine alternative Spanneinrichtung als Prinzipskizze gezeigt, mit der sich die Antriebsfeder 5 des Lanzettenhalters 7 und die Anschlagfeder 12 des Endanschlags 11 gleichzeitig spannen lassen. Sie umfaßt eine erste Zahnstange 32 und eine zweite Zahnstange 33, die über ein Zahnrad 34 miteinander verbunden sind. Die Zahnstange 32 ist mit dem nicht dargestellten Endanschlag gekoppelt. Die Zahnstange 33 ist mit dem Kopplungselement zwischen Lanzette und Antriebsfeder verbunden. Durch Bewegen der Zahnstange 32 wird auch die Zahnstange 33 indirekt über das Zahnrad 34 bewegt, so dass beim Spannen des Endanschlags auch indirekt die Lanzette gespannt wird. Die beiden Zahnstangen 32 und 33 ersetzen somit die Gewindestange 21 aus Figur 3.

Natürlich muß der Mitnehmer 26 des Lanzettenhalters 7 sowie der Endanschlag 11 aus Figur 3 an die Zahnstangen 32 bzw. 33 so angepaßt werden, dass die jeweiligen Mitnehmer aus dem Operationsweg des Endanschlags bzw. der Lanzette entfernt werden. Dies kann beispielsweise dadurch geschehen, dass die Zahnstangen jeweils an Blattfedern oder anderen elastischen Elementen Mitnehmer aufweisen und eine Rampe im Gestell bzw. im Gehäuse zum Beginn der Spannbewegung die Mitnehmer auslenkt. Nach Rückkehr in eine Neutralstellung der Zahnstangen 32 und 33 federn diese Mitnehmer aus dem Operationsweg von Lanzette und Endanschlag heraus.

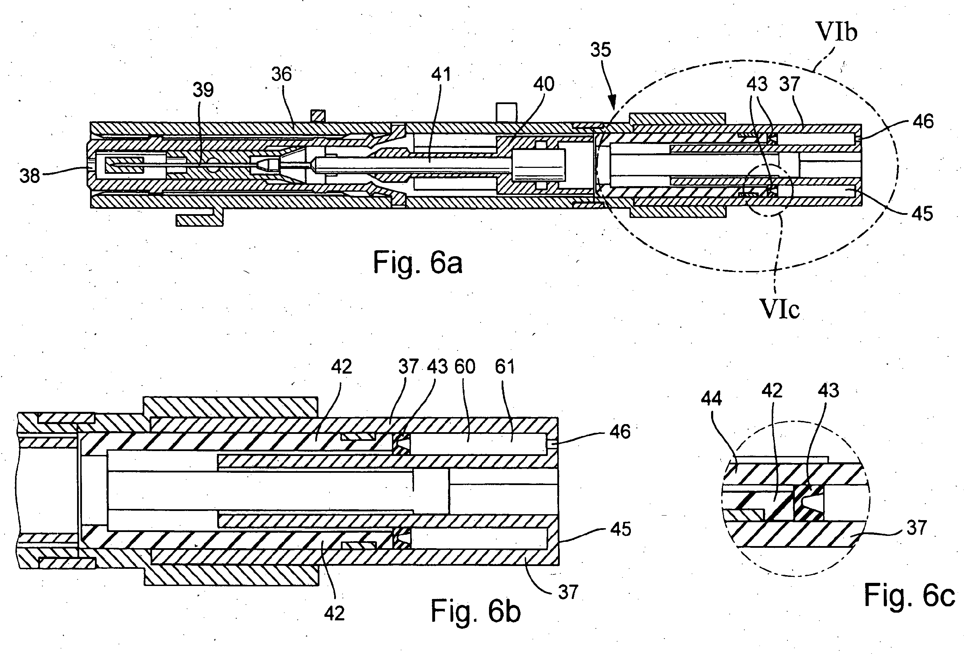

In den Figuren 6a,6b und 6c ist eine alternative Ausführungsform des erfindungsgemäßen Blutentnahmesystems gezeigt. Die Schwingungskontrolleinrichtung sind bei dieser Ausführungsform als Dämpfungseinrichtung 35 ausgeführt, durch das die Bewegung einer Lanzette 39 gedämpft und damit abgebremst wird. Das in Figur 6a gezeichnete Blutentnahmesystem weist ein Gehäuse 36 auf, das im wesentlichen zylindrisch ist. Es umfaßt einen Dämpfungszylinder 37, der das eine Ende des Gehäuses 36 bildet. Am entgegengesetzten Ende weist das Gehäuse 36 eine Öffnung 38 auf, durch die eine in dem Gehäuse 36 geführte Lanzette 39 austreten kann. Die Lanzette 39 ist mit einem Kopplungselement 40 gekoppelt, dass einen Antriebsstößel 41 umfaßt. Das Kopplungselement 40 ist mit einer nicht dargestellten Antriebsfeder verbunden, so dass die Lanzette 39 während der Stechbewegung mit der Antriebsfeder gekoppelt ist. Die Lanzette 39 wird von der Antriebsfeder während der Vortriebsphase und während der Rückführungsphase der Stechbewegung angetrieben.

Das Kopplungselement 40 weist an seinem von der Lanzette 39 abgewandten Ende einen Kolben 42 auf, der in der gezeigten Darstellung eine zylindrische Hülse mit dicker Wandstärke ist. Der Kolben 42 wird in einer Nut 60 geführt. Die Nut 60 ist im Dämpfungszylinder 37 angeordnet, der von seiner Rückwand 45 aus an betrachtet, einen doppelwandigen Zylinder darstellt. Die Nut 60 ist als Ringspalt ausgebildet, so dass der Kolben 42 zwischen einer Innenwand 44 des Dämpfungszylinders und der Außenwand des Dämpfungszylinders geführt wird. Am hinteren Ende des Kolbens 42 ist eine Dichtung angeordnet, die in Form einer doppelten Dichtlippe 43 ausgebildet ist. Die Dichtlippe besteht vorzugsweise aus einem gummiartigen Material, dass sowohl an der Außenwand des Dämpfungszylinders 37 als auch an der Innenwand 44 anliegt. Diese Dichtlippen 43 sind gegen die beiden Wände geneigt, so dass sie bei einer Vorwärtsbewegung des Kolbens 42 in Einstichrichtung, also während der Vortriebsphase der Stechbewegung der Lanzette 39, nur lose an der Innenwand 44 und am Dämpfungszylinder 37 anliegen. Die im Gehäuse 36 befindliche Luft kann nahezu ungeändert an den Dichtlippen 43 vorbeiströmen und wird in dem Dämpfungszylinder 37, insbesondere in einen Kompressionsraum 61 zwischen der Rückwand 45 und dem Kolben 42 hineingeleitet. Der Kompressionsraum 61 ist aufgrund der zylindrischen Ausführung des Gehäuses 36 als torusförmiger Raum gebildet. Der hülsenartig ausgebildete Kolben 42 kann genauso gut auch als einfacher Kolben ausgeprägt sein, der in einem einfachen Zylinder läuft. Die prinzipielle Arbeitsweise eines derartigen Kolbens und Zylinders ist mit der oben beschriebenen Weise gleich.

Nach Erreichen des Umkehrpunktes der Stechbewegung wird die Lanzette 39 durch die Antriebsfeder in der anschließenden Rückführungsphase der Stechbewegung wieder in das Gehäuse 36 zurückgezogen. Die leicht schräg stehenden Dichtlippen 43 werden während der Bewegung des Kopplungselementes 40 dicht an die Innenwand 44 und den Dämpfungszylinder 37 gedrückt. Die im torusförmigen Kompressionsraum 61 befindliche Luft kann nicht mehr zwischen den Dichtlippen 43 und der Innenwand 44 bzw. dem Dämpfungszylinder 37 hindurchströmen. Die Luft im Kompressionsraum 61 wird dadurch während, der Rückführungsphase der Stechbewegung komprimiert. Der Ringspalt bzw. die Nut 60 ist nahezu vollständig abgedichtet. Die während der Kompression entstehende Kompressionswärme wird an die angrenzenden Bauteile abgegeben.

Vorzugsweise ist in dem Kompressionsraum 61 an einer Rückwand 45 eine Ausströmöffnung 46 vorgesehen, durch die Luft entweichen kann. Der Durchmesser der Ausströmöffnung 46 ist jedoch so klein, dass es zu einer Komprimierung der im Dämpfungszylinder 37 befindlichen Luft kommt. Dadurch wird dem System Energie entzogen, insbesondere wird die Schwingungsenergie der Antriebsfeder der Lanzette 39 zur Komprimierung der Luft verbraucht. Die Bewegung der Lanzette 39 wird stark abgebremst. Durch geeignete Wahl der Geometrie und Größe des Dämpfungszylinders 37 sowie des Durchmessers der Ausströmöffnung 46 wird die Dämpfung des Gesamtsystems bestimmt. Der Durchmesser der Ausströmöffnung 46 kann vorteilhaft so gewählt werden, dass die Lanzette 39 schon während der ersten Rückführungsphase der Stechbewegung bis zum Stillstand abgebremst wird. Ein Nachschwingen der Lanzette 39 wird zuverlässig verhindert.

Die Ausströmöffnung 46 ist so zu dimensionieren, dass der Auslasswiderstand nicht zu klein ist, da sonst die Dämpfungswirkung gering ist. Andererseits darf der Strömungswiderstand nicht zu groß sein, weil sonst eher eine Luftfeder entsteht als eine Dämpfung. Die dabei anzupassenden Größen sind die bewegte Masse, die Federkonstante für Vortrieb und Rückführung, die Bewegungshübe, das Volumen des Dämpfungszylinders 37, der Strömungswiderstand der Ausströmöffnung 46 sowie der Reibfaktor der Dichtlippe 43 an der Innenwand 44 und dem Dämpfungszylinder 37. Diese Größen sind nicht alle frei wählbar; sie können aber experimentell so aufeinander abgestimmt werden, dass der gewünschte Effekt erzielt wird. Je größer z.B. die bewegte Masse ist, desto größer muß das Volumen im Dämpfungszylinder sein. Je länger der Bewegungshub, also die von der Lanzette 39 bzw. dem Kopplungselement 40 zurückgelegten Weg, desto kleiner sollte der Strömungswiderstand eingestellt werden.

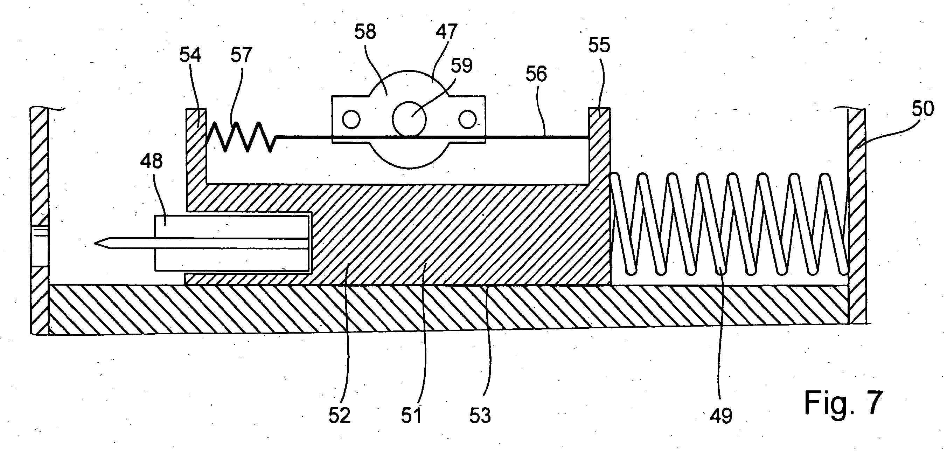

Figur 7 zeigt eine alternative Ausführungsform eines Dämpfungssystems als Prinzipskizze. Entgegen der Ausführung nach Figur 6a ist das Dämpfungssystem hier nicht als pneumatischer Dämpfer realisiert. In dem in Figur 7 gezeigten System arbeitet das Dämpfungssystem mit einem hydraulischen Dämpfer 47. Eine Lanzette 48 wird von einer Antriebsfeder 49, die mit einem Gehäuse 50 gekoppelte ist, angetrieben. Zwischen der Antriebsfeder 49 und der Lanzette 48 ist ein Kopplungselement 51 vorgesehen, dass als Lanzettenhalter 52 ausgebildet ist. Das Kopplungselement 51 wird an einer Linearführung 53 in einer geraden Bahn geführt, so dass die Stechbewegung während der Vortriebsphase und der Rückführungsphase eine Linearbewegung ist.

Das Kopplungselement 51 weist an seinem oberen und unteren Ende jeweils einen Steg 54 und 55 auf. Die beiden Stege 54 und 55 bilden mit der Seitenwand des Kopplungselements 51 ein U. Zwischen dem oberen Steg 54 und dem unteren Steg 55 ist ein Seil 56 gespannt, das direkt mit dem unteren Steg 55 verbunden. Zwischen dem oberen Steg 54 und dem Seil 56 ist eine Spannfeder 57 vorgesehen. Zwischen den beiden Stegen 54 und 55 ist der hydraulische Dämpfer 47 angeordnet, der als Rotationsdämpfer 58 ausgebildet ist. Der Rotationsdämpfer 58 hat eine Welle 59, um die das Seil 56 mit mehreren Umschlingungen umwickelt ist. Dadurch hat das Seil 56 gegen die Welle 59 des Rotationsdämpfers 58 einen Reibfaktor µ. Während der Bewegung des Kopplungselements 51 wird das Seil 56 durch die Spannfeder 57 mit einer Kraft F₀ stramm gezogen.

Wird das Kopplungselement 51 aus der gespannten Position (also der Position, in der die Antriebsfeder 49 gespannt ist) freigelassen, so treibt die Antriebsfeder 49 das Kopplungselement 51 und die Lanzette 48 nach vorne, so dass die Spitze der Lanzette 48 aus einer Öffnung des Gehäuses 50 des Blutentnahmesystems austreten kann.

Während der Vortriebsphase der Stechbewegung der Lanzette 48 wird das hintere Ende des Seils 56 entlastet, während die Spannfeder 57 weiter gespannt wird. Dadurch sinkt die Zugkraft an dem Seil 56 auf einen minimalen Wert ab. Dies hat zur Folge, dass das Seil 56 während der Vortriebsphase der Stechbewegung der Lanzette 48 auf der Welle 59 des Rotationsdämpfers 58 durchrutscht und nur mit einer sehr kleinen Reibkraft gebremst wird.

Nach Erreichen des Umkehrpunktes der Stechbewegung wirkt durch den unteren Steg 55 des Kopplungselementes 51 eine Zugkraft auf das hintere Ende des Seils 56. Dabei hält die Spannfeder 57 das Seil 56 straff. Damit kann eine Kraft F' = F₀ · e^{µα} auf die Welle 59 des Dämpfers übertragen werden, wobei α den Umschlingungswinkel angibt, der sich aus der Anzahl der Umschlingungen des Seils 56 um die Welle 59 des Rotationsdämpfers 58 multipliziert mit 360° ergibt. Wenn die Spannfeder 57 entsprechend dimensioniert ist, dreht sich die Welle 59 des Rotationsdämpfers 58 mit der Bewegung des Kopplungselements 51 während der Rückführungsphase der Stechbewegung mit und bremst damit die Bewegung der Lanzette 48 auf eine gewünschte, vorbestimmte geringe Geschwindigkeit ab. Damit wird ein Nachschwingen des Kopplungselements 51 durch die Antriebsfeder 49 zuverlässig vermieden. Ein Wiederaustritt der Lanzette 48 aus dem Gehäuse ist nicht möglich; ein Mehrfachstechen des Körperteils ist ausgeschlossen.

## Patentansprüche

1. Blutentnahmesystem zur Entnahme von Blut aus einem Körperteil für Diagnosezwecke, umfassend
ein Gehäuse (2,36,50) mit einer Lanzettenführung (3), von der eine Lanzette (8,39,48) auf einem vorbestimmten Einstichweg geführt wird, und
einen Lanzettenantrieb (4) zum Antreiben einer Stechbewegung der Lanzette (8,39,48) auf dem vorbestimmten Einstichweg,
wobei
die Stechbewegung eine Vortriebsphase in Einstichrichtung und nach Erreichen eines Umkehrspunkts eine anschließende Rückführungsphase entgegen der Einstichrichtung umfaßt, und
der Lanzettenantrieb (4) eine Antriebsfeder (5,49) aufweist, die hinter der Lanzette (8,39,48) angeordnet ist und während der Vortriebsphase der Stechbewegung so mit der Lanzette (8,39,48) verbunden ist, dass die Lanzette (8,39,48) in Einstichrichtung angetrieben wird,
**dadurch gekennzeichnet, dass**
eine Schwingungskontrolleinrichtung, die eingerichtet ist, auf ein die Lanzette (8,39,48) und die Antriebsfeder (5,49) einschließendes schwingunsfähiges System so einzuwirken, dass dessen Schwingungsverhalten geändert und dadurch ein mehrfaches Einstechen der Lanzette (8,39,48) in das Körperteil verhindert wird, von oben
die Schwingungskontrolleinrichtung eingerichtet ist, im wesentlichen nur während der Rückführungsphase der Stechbewegung auf die Lanzette (8,39,48) einzuwirken.

2. Blutentnahmesystem nach Anspruch 1, **dadurch gekennzeichnet, dass** die Schwingungskontrolleinrichtung einen bewegbaren Endanschlag (11) umfasst, der nach Erreichen des Umkehrpunkts der Stechbewegung der Lanzette (8,39,48) entgegen der Einstichrichtung in eine Halteposition verschoben wird.

3. Blutentnahmesystem nach Anspruch 2, **dadurch gekennzeichnet, dass** der Endanschlag (11) in seiner Halteposition fixiert wird.

4. Blutentnahmesystem nach Anspruch 2, **dadurch gekennzeichnet, dass** der Endanschlag (11) in seiner Halteposition elastisch gehalten wird.

5. Blutentnahmesystem nach einem der Ansprüche 2 bis 4, **dadurch gekennzeichnet, dass** der Endanschlag (11) durch eine Anschlagfeder (12) in seine Halteposition verschoben wird.

6. Blutentnahmesystem nach Anspruch 5, **dadurch gekennzeichnet, dass** die Anschlagfeder (12) während der Stechbewegung in Einstichrichtung der Lanzette (8,39,48) vorgespannt und arretiert ist und die Arretierung am Ende der Vortriebsphase der Stechbewegung der Lanzette (8,39,48) gelöst wird.

7. Blutentnahmesystem nach Anspruch 5 oder 6, **dadurch gekennzeichnet, dass** die Anschlagfeder (12) eine Restspannung aufweist, wenn der Endanschlag (11) in seine Halteposition verschoben ist.

8. Blutentnahmesystem nach einem der Ansprüche 5 bis 7, **dadurch gekennzeichnet, dass** zum Spannen der Anschlagfeder (12) eine Spannvorrichtung (20) vorgesehen ist, die eine Gewindestange (21) mit Gewinde (24) und ein Spannelement (22) aufweist, das durch Drehen der Gewindestange (21) bewegt wird, wobei das Spannelement (22) während seiner Bewegung zum Spannen der Anschlagfeder (12) an einer Führungsfläche (30) geführt wird.

9. Blutentnahmesystem nach Anspruch 8, **dadurch gekennzeichnet, dass** die Gewindestange (21) zwei gegenläufige Gewinde (24,25) aufweist und jedes Gewinde ein Spannelement (22,23) bewegt, wobei das erste Spannelement (22) die Anschlagfeder (12) und das zweite Spannelement (23) die Antriebsfeder (5) spannt, so dass die Anschlagfeder (12) und die Antriebsfeder (5) gleichzeitig gespannt werden.

10. Blutentnahmesystem nach Anspruch 9, **dadurch gekennzeichnet, dass**, das erste Gewinde (24) und das zweite Gewinde (25) der Gewindestange (21) unterschiedliche Steigungen aufweisen.

11. Blutentnahmesystem nach einem der Ansprüche 8 bis 10, **dadurch gekennzeichnet, dass** das Spannelement (22) beim Bewegen entgegen der Bewegrichtung zum Spannen an einer Rückleitkante (31) so geführt wird, dass das Spannelement (22) mit der Anschlagfeder (12) außer Eingriff kommt.

12. Blutentnahmesystem nach Anspruch 1, **dadurch gekennzeichnet, dass** die Schwingungskontrolleinrichtung eine Dämpfungseinrichtung (35) einschließt, durch das die Bewegung der Lanzette (8,39,48) in der Rückführungsphase abgebremst wird.

13. Blutentnahmesystem nach Anspruch 12, **dadurch gekennzeichnet, dass** die Dämpfungseinrichtung (35) einen hydraulischen Dämpfer (47) aufweist.

14. Blutentnahmesystem nach Anspruch 12, **dadurch gekennzeichnet, dass** die Dämpfungseinrichtung (35) einen pneumatischen Dämpfer aufweist.

15. Blutentnahmesystem nach Anspruch 14, **dadurch gekennzeichnet, dass** ein Kopplungselement (6,40,51) zwischen der Lanzette (8,39,48) und der Antriebsfeder (49) vorgesehen ist, welches in einem Zylinder geführt wird, und dass an dem Kopplungselement (40) Dichtlippen (43) vorgesehen sind, die an der Innenwand (44) des Zylinders anliegen.

16. Blutentnahmesystem nach Anspruch 15, **dadurch gekennzeichnet, dass** die Dichtlippen (43) des Kopplungselements (40) während der Vortriebsphase der Stechbewegung der Lanzette (39) an der Innenwand (44) des Zylinders so anliegen, dass die Lanzette (39) nicht wesentlich gebremst wird, und während der Rückführungsphase der Lanzette (39) dichtend an der Innenwand (44) des Zylinders derartig geführt werden, dass die während der Rückführungsphase vor den Dichtlippen (43) in dem Zylinder enthaltene Luft komprimiert wird, wodurch die Lanzette (39) während der Rückführungsphase abgebremst wird.

## Claims

1. Blood collection system for collecting blood from a body part for diagnostic purposes, comprising
a housing (2, 36, 50) with a lancet guide (3) by which a lancet (8, 39, 48) is guided on a predetermined puncture path, and
a lancet drive (4) for driving a puncture movement of the lancet (8, 39, 48) on the predetermined puncture path,
wherein
the puncture movement comprises a forward movement phase in the direction of puncturing and after reaching a reversal point, a subsequent retraction movement phase in the direction opposite the puncture direction, and
the lancet drive (4) has a driving spring (5, 49) which is arranged behind the lancet (8, 39, 48) and during the forward movement phase of the puncturing movement is connected to the lancet (8, 39, 48) in such a manner that the lancet (8, 39, 48) is driven in the puncturing direction,
an oscillation control device arranged for controlling oscillation of an oscillatory system, comprising the lancet (8, 39, 48) and the driving spring (5, 49), in such a manner that the oscillation behavior of the oscillatory system is altered whereby a repeated puncturing of the body part by the lancet (8, 39, 48) is prevented
**characterized in that**
the oscillation control device is arranged in order to act on the lancet (8, 39, 48) essentially only during the retraction movement phase of the puncturing movement.

2. Blood collection system according to Claim 1, **characterized in that** the oscillation control device comprises a movable end stop (11) which is displaced into a holding position in the direction opposite the puncturing direction after the lancet (8, 39, 48) has reached the reversal point to its puncturing movement.

3. Blood collection system according to Claim 2, **characterized in that** the end stop (11) is secured in its holding position.

4. Blood collection system according to Claim 2, **characterized in that** the end stop (11) is kept flexible in its holding position.

5. Blood collection system according to any one of the Claims 2 to 4, **characterized in that** the end stop (11) is displaced into its holding position by a stop spring (12).

6. Blood collection system according to Claim 5, **characterized in that** the stop spring (12) is prestressed and locked during the puncturing movement of the lancet (8, 39, 48) in the puncturing direction, and **in that** the lock is released at the end of the forward movement phase of the puncturing movement of the lancet (8, 39, 48).

7. Blood collection system according to Claim 5 or 6, **characterized in that** the stop spring (12) has a residual tension when the end stop (11) has been displaced into its holding position.

8. Blood collection system according to any one of the Claims 5 to 7, **characterized in that** a tension device (20) for applying tension to the stop spring (12) is provided, said tension device (20) comprising a threaded rod (21) with a thread (24) and a tension element (22) that is moved by rotation of the threaded rod (21), wherein the tension element (22) is guided on a guide face (30) during its movement to apply tension to the stop spring (12).

9. Blood collection system according to Claim 8, **characterized in that** the threaded rod (21) has two threads (24, 25) running in opposite directions and each thread moving a tension element (22, 23), wherein the first tension element (22) applies tension to the stop spring (12) and the second tension element (23) applies tension to the driving spring (5) so that the stop spring (12) and the driving spring (5) are put under tension simultaneously.

10. Blood collection system according to Claim 9, **characterized in that** the first thread (24) and the second thread (25) of the threaded rod (21) have different pitches.

11. Blood collection system according to any one of the Claims 8 to 10, **characterized in that** the tension element (22) is guided on a return edge (31) in its movement opposite the direction of movement for applying tension in such a manner that the tension element (22) becomes disengaged with the stop spring (12).

12. Blood collection system according to Claim 1, **characterized in that** the oscillation control device comprises a damping device (35) by means of which the movement of the lancet (8, 39, 48) is decelerated in the retraction movement phase.

13. Blood collection system according to Claim 12, **characterized in that** the damping device (35) comprises a hydraulic damper (47).

14. Blood collection system according to Claim 12, **characterized in that** the damping device (35) comprises a pneumatic damper.

15. Blood collection system according to Claim 14, **characterized in that** a coupling element (6, 40, 51) is provided between the lancet (8, 39, 48) and the driving spring (49), said coupling element (6, 40, 51) being guided in a cylinder, and **in that** sealing lips (43) are provided on the coupling element (40), said sealing lips (43) being in contact with the inner wall (44) of the cylinder.

16. Blood collection system according to Claim 15, **characterized in that** during the forward movement phase of the puncturing movement of the lancet (39) the sealing lips (43) of the coupling element (40) touch the inner wall (44) of the cylinder in such a manner that the lancet (39) is not significantly decelerated, and during the retraction movement phase of the lancet (39) the sealing lips (43) are in sealing contact with the inner wall (44) of the cylinder in such a manner that the air contained in the cylinder in front of the sealing lips (43) during the retraction movement phase is compressed, whereby the lancet (39) is decelerated during the retraction movement phase.

## Revendications

1. Système de prélèvement de sang pour prélever du sang sur une partie du corps à des fins de diagnostic, comprenant
un boîtier (2, 36, 50) avec un guide de lancette (3) par lequel une lancette (8, 39, 48) est guidée sur un parcours de piqûre prédéterminé, et
un entraînement de lancette (4) pour entraîner un mouvement de piqûre de la lancette (8, 39, 48) sur le parcours de piqûre prédéterminé,
ledit mouvement de piqûre comprenant une phase d'avancement dans le sens de la piqûre et, après avoir atteint un point d'inversion, une phase de recul en sens inverse de la piqûre, et
ledit entraînement de lancette (4) étant muni d'un ressort d'entraînement (6, 49), disposé derrière la lancette (8, 39, 48) et relié à la lancette (8, 39, 48) pendant la phase d'avancement du mouvement de piqûre de façon à entraîner la lancette (8, 39, 48) dans le sens de la piqûre,
un moyen de commande d'oscillation, adapté pour agir sur un système oscillant incluant la lancette (8, 39, 48) et le ressort d'entraînement (5, 49) de façon à modifier le comportement oscillant dudit système et à empêcher ainsi la lancette (8, 39, 48) de piquer plusieurs fois la partie du corps,
**caractérisé en ce que**
le moyen de commande d'oscillation est adapté pour agir sur la lancette (8, 39, 48) essentiellement uniquement pendant la phase de recul.

2. Système de prélèvement de sang selon la revendication 1, **caractérisé en ce que** le moyen de commande d'oscillation comprend une butée de fin de course mobile (11) qui, une fois atteint le point d'inversion du mouvement de piqûre de la lancette (8, 39, 48), est déplacée en sens inverse de la piqûre dans une position d'immobilisation.

3. Système de prélèvement de sang selon la revendication 2, **caractérisé en ce que** la butée de fin de course (11) est fixée dans sa position d'immobilisation.

4. Système de prélèvement de sang selon la revendication 2, **caractérisé en ce que** la butée de fin de course (11) est maintenue élastiquement dans sa position d'immobilisation.

5. Système de prélèvement de sang selon l'une des revendications 2 à 4, **caractérisé en ce que** la butée de fin de course (11) est déplacée dans sa position d'immobilisation par un ressort de butée (12).

6. Système de prélèvement de sang selon la revendication 5, **caractérisé en ce que** le ressort de butée (12) est précontraint et bloqué pendant le mouvement de piqûre dans le sens de la piqûre de la lancette (8, 39, 48) et est débloqué à la fin de la phase d'avancement du mouvement de piqûre de la lancette (8, 38, 48).

7. Système de prélèvement de sang selon la revendication 5 ou 6, **caractérisé en ce que** le ressort de butée (12) présente une contrainte résiduelle lorsque la butée de fin de course (11) est déplacée dans sa position d'immobilisation.

8. Système de prélèvement de sang selon l'une des revendications 5 à 7, **caractérisé en ce qu'**un dispositif tendeur (20) pour tendre le ressort de butée (12) est prévu, qui comprend une tige filetée (21) munie d'un filetage (24) et un élément tendeur (22) se déplaçant par rotation de la tige filetée (21), l'élément tendeur (22) étant guidé pendant son déplacement sur une surface de guidage (30) pour tendre le ressort de butée (12).

9. Système de prélèvement de sang selon la revendication 8, **caractérisé en ce que** la tige filetée (21) est munie de deux filetages opposés (24, 25) et chaque filetage déplace un élément tendeur (22, 23), le premier élément tendeur (22) venant tendre le ressort de butée (12) et le second élément tendeur (23) le ressort d'entraînement (5), de telle sorte que le ressort de butée (12) et le ressort d'entraînement (5) sont tendus simultanément.

10. Système de prélèvement de sang selon la revendication 9, **caractérisé en ce que** le premier filetage (24) et le second filetage (25) de la tige filetée (21) ont des pas différents.

11. Système de prélèvement de sang selon l'une des revendications 8 à 10, **caractérisé en ce que** l'élément tendeur (22), en se déplaçant en sens inverse du sens de déplacement permettant de le tendre, est guidé sur une arête de renvoi (31) de façon à désengager l'élément tendeur (22) du ressort de butée (12).

12. Système de prélèvement de sang selon la revendication 1, **caractérisé en ce que** le moyen de commande d'oscillation inclut un moyen d'amortissement (35) par lequel le mouvement de la lancette (8, 39, 48) est freiné dans la phase de recul.

13. Système de prélèvement de sang selon la revendication 12, **caractérisé en ce que** le moyen d'amortissement (35) comprend un amortisseur hydraulique (47).

14. Système de prélèvement de sang selon la revendication 12, **caractérisé en ce que** le moyen d'amortissement (35) comprend un amortisseur pneumatique.

15. Système de prélèvement de sang selon la revendication 14, **caractérisé en ce qu'**un élément de couplage (6, 40, 51) est prévu entre la lancette (8, 39, 48) et le ressort d'entraînement (49), qui est guidé dans un cylindre, et **en ce que** des lèvres d'étanchéité (43) sont prévues sur l'élément de couplage (40), qui s'appliquent contre la paroi interne (44) du cylindre.

16. Système de prélèvement de sang selon la revendication 15, **caractérisé en ce que** les lèvres d'étanchéité (43) de l'élément de couplage (40) s'appliquent contre la paroi interne (44) du cylindre pendant la phase d'avancement du mouvement de piqûre de la lancette (39) de façon à ne pas freiner la lancette (39) d'une manière sensible, et sont guidées de manière étanche sur la paroi interne (44) du cylindre pendant la phase de recul de la lancette (39) de façon à comprimer l'air contenu dans le cylindre devant les lèvres d'étanchéité (43) pendant la phase de recul, ce qui freine la lancette (39) pendant la phase de recul.
